Europäisches Patentamt

(19) European Patent Office

Office européen des brevets

(11) Publication number: **0 363 125**

**A2**

(12) **EUROPEAN PATENT APPLICATION**

(21) Application number: **89310059.4**

(51) Int. Cl.5: **C12N 5/00**

(22) Date of filing: **02.10.89**

(30) Priority: **03.10.88 US 252300**

(43) Date of publication of application:
**11.04.90 Bulletin 90/15**

(84) Designated Contracting States:
**AT BE CH DE ES FR GB GR IT LI LU NL SE**

(71) Applicant: **HANA BIOLOGICS INC.**
**850 Marina Village Parkway**
**Alameda California 94501(US)**

(72) Inventor: **Zayas, Julie Robinson**
**1370 3rd Street No.115**
**Alameda California 94501(US)**
Inventor: **Voss, Houston Fredrick**
**604 Windmill Lane**
**Pleasanton California 94566(US)**
Inventor: **Walthall, Bennie Joe**
**10431 Boca Canyon Drive**
**Santa Ana California 92750(US)**
Inventor: **Moss, Paul Stewart**
**287 Lake Drive**
**Kensington California 94708(US)**
Inventor: **McHugh, Yvonne E.**
**2115 Los Angeles Avenue**
**Berkeley California 94707(US)**

(74) Representative: **Harrison, David Christopher**
**et al**
**MEWBURN ELLIS 2 Cursitor Street**
**London EC4A 1BQ(GB)**

(54) **Proliferated pancreatic endocrine cell product and process.**

(57) Proliferated human fetal pancreatic cells comprising a mixture containing proliferated human fetal pancreatic islet progenitor cells, the proportion of pancreatic islet progenitor cells to exocrine and fibroblast cells being substantially greater than in fetal pancreas. The mixture can be in a culture medium, having pancreatic islet progenitor cells on or dispersed in a substrate matrix which can include collagen. It should be substantially free of fibroblast cells (preferably less than 10 percent), and can be in the form of droplets or other shapes suitable for implantation.

These progenitor cells are prepared by culturing human fetal pancreatic cells containing pancreatic islet cells, pancreatic islet progenitor cells, exocrine cells, and fibroblast cells; separating the epithelial cells containing the islet progenitor cells from the fibroblasts by subculture; and proliferating the pancreatic islet progenitor cells through one or more subcultures until the relative proportion of the pancreatic islet progenitor cells to fibroblast and exocrine cells and the total number of pancreatic islet progenitor cells is substantially greater than in the original fetal pancreas.

## PROLIFERATED PANCREATIC ENDOCRINE CELL PRODUCT AND PROCESS

This invention relates to preparation of proliferated human fetal pancreatic islet progenitor cells and their implantation. In particular, this invention relates to processes for producing proliferated human islet progenitor cells which, when implanted in humans having missing or deficient pancreatic β-cells, a characteristic of diabetes mellitus, are capable of developing into functional islets containing mature islet cells.

Restoration of pancreatic β-cell function in persons having an insufficiency of these cells has been an object of extensive research. Implantation of pancreatic tissue appears to be a promising approach, as implants of pancreatic tissue fragments in mice have been shown to restore their pancreatic function and reduce blood glucose to normal levels. Immune reactions and ultimate rejection of the implanted pancreatic tissue remain serious problems. Acceptable large-scale sources for suitable pancreatic tissue have yet to be developed. Laboratory experiments have relied upon the use of cells and/or tissue from adult or fetal cadavers. As used herein, the following terms are defined as follows:

A "mature islet cell" is a mature endocrine cell capable of secreting hormones in response to specific secretagogues. For example, a mature pancreatic islet beta cell is capable of secreting insulin in response to glucose stimulation. A mature islet cell is found in an adult pancreas, and usually contains significant intracellular stores of a specific hormone which can be detected by immunohistochemical staining techniques. For example, a mature pancreatic islet beta cell will stain positively for insulin; a mature pancreatic islet alpha cell will stain positively for glucagon.

"Immature islet cells" are found in the human fetal pancreas starting at about 8 weeks of gestation. An immature islet cell contains significant intracellular levels of a specific hormone (e.g., insulin in pancreatic islet beta cells, glucagon in pancreatic islet alpha cells), and will stain positively immunohistochemically for that hormone. An immature pancreatic islet beta cell is not capable of responding to glucose stimulation, although it can release insulin *in vitro* in response to secretagogues such as theophylline and forskolin, which increase intracellular cAMP levels.

An "islet progenitor cell" is a cell of endocrine lineage found in the fetal pancreas. It does not contain intracellular islet hormones, and does not respond to secretagogues. It is capable of differentiating into an immature islet cell, and finally into a mature islet cell, under correct environmental stimuli, such as after transplantation.

Islet progenitor cells belong to a class of cells known as "epithelial cells". Epithelial cells can be distinguished from fibroblasts by their characteristic cell shape. Epithelial cells are cuboidal or polygonal, whereas fibroblasts are elongated or spindle shaped.

"Cell culture" or "tissue culture" refers to the maintenance of cell viability and function *in vitro*. It may or may not involve cell proliferation.

"Cell proliferation" is a process of cell multiplication by means of cell division. When this occurs *in vitro* to any significant level, it usually involves one or more subcultures.

Methods have been developed for preparing *in vitro* primary cultures of human fetal pancreatic tissue and/or cells. Sandler et al., "Tissue culture of human fetal pancreas: development and function of β-cells *in vitro* and transplantation of explants to nude mice." *Diabetes.* 34:1113-1119 (1985) describes the preparation of insulin-producing human cell clusters from fetal pancreatic tissue obtained by prostaglandin-induced abortions. The tissue was partially disintegrated with collagenase, and maintained in a culture medium. These cell clusters were implanted beneath the kidney capsule of nude mice, and examination of the implants in sacrificed animals showed the presence of islet cells that stained positive for insulin and glucagon. Selective proliferation of fetal pancreatic islet progenitor cells is not disclosed. Agen et al., "Human fetal pancreas: culture and function *in vitro*." *Diabetes.* 29:64-69 (1980) describes in vitro culture studies with human fetal pancreas tissue which show that immature islet cells may retain viability in organ culture for more than a week. Rabinovitch et al., "Growth hormone stimulates islet β-cell replication in neonatal rat pancreatic monolayer cultures." *Diabetes,* 32:307-312 (1983) discloses that growth hormone may have value in stimulating mature β-cell replication *in vitro* in pancreatic monolayer cultures. Nielsen, in "Effects of growth hormone, prolactin, and placental lactogen on insulin content and release, and deoxyribonucleic acid synthesis in cultured pancreatic islets" *Endocrinology.* 110:600-606 (1982) reports *in vitro* culture studies with pancreatic tissue from adult NMRI mice and adult or newborn Wistar rats and the significant increase in insulin content and evidence of possible increased deoxyribonucleic acid synthesis observed with these additives. Fong et al., "Hormones and factors that stimulate growth of a rat islet tumor cell line in serum-free medium." *Diabetes.* 30:1022-1028 (1981) report elucidation of substances required for growth in serum-free medium of pancreatic islet tumor cells. They found the these cells could be grown in a serum-free hormone- and nutrient-defined medium in which the ability to produce insulin is preserved. Swenne et al., "Growth hormone regulation of Somatomedin C/insulin-like growth of Factor I production and DNA replication in fetal rat islets in tissue culture." *Diabetes.* 36:288-294 (1987) report that glucose-stimulated cell growth of rat immature islet cells of digested fetal rat pancreatic tissue was markedly reduced in serum containing less than one percent fetal calf serum.

Requirements for primary cell culture of pancreatic $\beta$-cells is greatly influenced by the substrate upon which the cells are grown. Thivolet et al., "Morphological and functional effects of extracellular matrix on pancreatic islet cell cultures." *Exp.Cell Res.* 159:313-322 (1985) reports that extracellular matrix (ECM) enhances mature rat islet cell attachment and proliferation as well as inducing differentiation *in vitro.* Pancreatic tissue from one-month old Sprague-Dawley rats was treated to isolate mature islets and to produce isolated cells. Floating mature islet cells were seeded onto dishes coated with plastic or ECM. Some initial attachment to the plastic was observed, but the cells usually detached from the plastic between days 7 and 10. On ECM, colonies attached and formed quickly. The colonies remained well preserved up to 15 days, and fibroblast overgrowth was delayed. Hirata et al., "Duct, exocrine, and endocrine components of cultured fetal mouse pancreas." *In Vitro.* 18:789-798 (1982) report studies with twenty to twenty-two days postcoitum mouse fetal pancreas organ bits cultured on the dermal surface of irradiated pigskin as a substrate. Cell growth could only be continued if the enzymatic destruction of the pigskin was prevented by adding soybean trypsin inhibitor or atropine to the culture medium.

Fedotov et al., *Biull.Esksper.Biol.* 8:235 (1978), Shumakov et al,*Biull.Esksper.Biol.* 8:202 (1979), and Bliumkin et al., *Biull.Esksper.Biol.* 95:89 (1983) report research to develop improved *in vitro* primary cultures of immature islet cells.

Successful implantation in animals was reported by Bliumkin et al., *Biull.Esksper.Biol.* 95:89 (1983), who reported that implantation of primary culture cells in rats having experimentally induced diabetes yielded a reduction in serum glucose level by day five to day seven.

Successful human implantations have also been reported. Shumakov et al., *Probl.Endokrin.* 31:67 (1985) report successful implantation of fetal human or porcine pancreatic primary culture cells in humans with advanced diabetes. Many of the patients receiving the implants had dramatic, rapid and sustained remissions of diabetic symptoms and pathology for extended times. Shumakov et al., *Klin.Med* 61:46 (1983) reported successful results with human diabetics implanted with human fetal pancreas primary culture cells.

U.S. Patent 4,495,288 describes a capsular membrane system for culturing pancreatic $\beta$-cells, $\alpha$-cells and $\delta$-cells.

U.S. Patent 4,485,096 (column 5, lines 63-68) describes a procedure for supporting cells on or in a contracted collagen structure, listing insulin producing pancreatic cells among the cells which can be added. The collagen structure is made by contracting a collagen solution containing Collagen Type I with a contractile agent. The contractile agents are cells such as fibroblast cells, smooth muscle cells, striated muscle cells, and heart muscle cells which adhere to Collagen Type I fibers and pull them together into a more compact structure. This process is said to be accelerated by adding thrombin or other platelet release factors such as fatty acids, ADP and $\gamma$-globulin.

U.S. Patents 4,539,716 and 4,546,500 describe the preparation of living blood vessels and glandular tissues using a contracted collagen structure, and lists pancreatic cells (islets) as possible cells for lining this structure, in an effort to provide an artificial organ.

The previously reported human fetal pancreatic culture products developed and used in implantation experiments with humans and animals were obtained from primary cultures containing a mixture of immature islet cells, islet progenitor cells, and other cells found in the fetal pancreas. In the prior art procedures for selective concentration of pancreatic $\beta$-cells, the immature islet cells do not significantly proliferate; they generally age and ultimately die. Therefore, only a small percentage of the immature islet cells in the original pancreas tissue survive for implantation.

This invention is based on the development of subculture procedures for culturing and proliferating human fetal pancreas islet progenitor cells, and is directed to growth, storage, production and implantation of proliferated, human fetal pancreas islet cells. The subculturing is designed to increase the number of human fetal progenitor cells in a population and to minimize the proportion of undesirable cells, such as exocrine and fibroblast cells. The number of islet progenitor cells obtained far exceeds the number in the original fetal tissue. The islet progenitor cells in the proliferated cultures do not produce insulin, may have greatly reduced antigenicity, and yield insulin producing islets *in vivo* after implantation by the natural process of *in vivo* cell growth and differentiation.

The products of this invention comprise a mixture containing proliferated human fetal islet progenitor cells, the proportion of pancreatic islet progenitor cells to exocrine-lineage and fibroblast cells being substantially greater than in natural fetal pancreas tissue. The mixture can be cells or aggregates of cells in a culture medium, or single or aggregated pancreatic islet progenitor cells on or dispersed in a substrate matrix. It should be substantially free of fibroblast cells (i.e. should contain not more than 15-20% and preferably less than 10%), and can be in the form of droplets or other shapes suitable for implantation. In a preferred embodiment, the matrix can be contracted collagen or a contractible mixture of proliferated human fetal pancreatic islet progenitor cells in a matrix of Collagen Type I containing laminin and Collagen Type IV.

The process of this invention for preparing proliferated human fetal pancreatic islet progenitor cells comprises obtaining human fetal pancreatic tissue from a donor; digestion of the tissue to obtain cell clusters for culture; culturing the cells thus obtained to decrease the number of fibroblast cells and exocrine cells; and culturing the human fetal pancreatic islet progenitor cells to increase their number. More specifically, the process involves a primary culture containing human fetal pancreatic cells; subculture to separate the epithelial cells from the fibroblasts; and proliferation of the epithelial cells through one or more subcultures, until the total number of pancreatic islet progenitor cells is greater than in the original fetal pancreatic tissue.

## DETAILED DESCRIPTION

Culture techniques for preparing fetal pancreatic cells for implantation have focused on isolating immature islet cells from human fetal pancreatic tissue. Immature islet cells have a very limited capacity to proliferate. The application of implantation therapy to persons experiencing diabetes mellitus requires a reliable supply of suitable cells. This supply is dependent upon obtaining the requisite number of cells from adult or fetal cadavers, and maintaining cell viability until implantation. Isolation and primary culture of human fetal pancreatic cells from fetal cadavers is a limited source.

This invention is based on the discovery that undifferentiated pancreatic islet progenitor cells can be proliferated in subculture, providing a product mixture which is greatly enriched in the desired cell type, thus providing a reliable and uniform supply of the cells. The pancreatic islet progenitor cells, when suitably implanted, differentiate *in vivo* to form insulin-producing pancreatic islet structures at the implantation site. The pancreatic islet progenitor cells in the implant grow and differentiate into mature pancreatic islets, to become a functioning source of insulin, glucagon and somatostatin for regulation of blood glucose levels in the patient.

Key steps for culturing human fetal pancreas (HFP) islet progenitor cells are outlined below. While the steps are provided in detail by way of example for purposes of clarity, it will be readily apparent to a person skilled in the art that the procedures described can be modified and varied without departing from the objective thereof, and this invention is not limited to the specific details presented herein.

## TISSUE PREPARATION

The pancreas is aseptically dissected from the fetus and placed in a sealed container with cold (0-8° C) shipping medium. A suitable medium is L-15 (Liebovitz) medium supplemented with 5% fetal bovine serum (FBS) and antibiotics. The optimal time lapse between dissection and commencement of processing for culture should be less than 4 hours. Times longer than this result in cold ischemia and loss of cell viability. Cold ischemia times of 24 hours or more result in a 50-95% loss of cell viability.

At the processing site, the pancreas is removed from the shipping medium and dissected away from any adherent connective tissue (including the capsule and major interlobular laminae). Cleaning of the tissues is done on ice or under other refrigeration. The cleaned pancreas is chopped into 0.5-2.0 mm blocks. The tissue blocks are then digested at 37° C using a collagenase enzyme solution and mechanical shaking. An optimal collagenase solution contains 0.1-2.0 mg/ml of Collagenase Type XI (Sigma) (highly purified, with a minimum of 1200 Units per mg of solid); 0.01-0.5 mg/ml of Deoxyribonuclease Type II (approximately 2000 kunitz units per mg solid); 0.1-2.0 wt.% polyvinylpyrrolidone (PVP-40,Sigma), made in Hank's Balanced Salt Solution (HBSS) containing 0.15% HSA, pH 7.4.

The collagenase enzyme should have low levels of contaminating protease activity (especially trypsin) because these proteases cause excessive cell damage and lysis during the digestive procedure. Human serum albumin (HSA) is added to the enzyme solution to absorb some of the non-specific protease activity. Deoxyribonuclease will degrade DNA released as a result of cell lysis and thus prevent entrapment of cells

4

in DNA. PVP-40 is added to assist in protecting the cells from damage during the digestion procedure.

The objective of the digestion procedure is to disperse the tissue into small cell aggregates (20-2000 cells per aggregate) without prolonged exposure to the digestive enzymes. Sequential incubations of the tissue blocks in the enzyme solution are carried out in 5-20 minute intervals. At the end of each incubation period, small cell aggregates that have been released from the tissue pieces are decanted off. The process is repeated until the tissue pieces are completely digested into small cell aggregates.


## PROLIFERATION


An optimal (serum-free) culture medium for HFP islet progenitor cells contains a basal nutrient medium such as Medium M199 (available from Mediatech, catalog no. 10-060-IV). The medium is suitably buffered, for example with 26 mM $NaHCO_3$ and 10-30 mM HEPES (N-2-Hydroxyethylpiperazine-N'-2-ethanesulfonic acid), pH 7.4. The medium is supplemented with antibiotics and the growth factors and hormones shown in Table A.

TABLE A

| Component | Concentration |
|---|---|
| Bovine Pituitary Extract (BPE) | 1-40 $\mu$g/ml |
| Insulin | 1-10 $\mu$g/ml |
| Transferrin | 1-20 $\mu$g/ml |
| High Density Lipoprotein (HDL) | 10-500 $\mu$g/ml |
| Growth Hormone (GH) | 0.01-1 $\mu$g/ml |
| Epidermal Growth Factor (EGF) | 1-50 ng/ml |
| Glucagon | 0.1-1 $\mu$g/ml |
| Triiodothyronine ($T_3$) | 0.001-1 ng/ml |
| Hydrocortisone | 0.1-1 $\mu$g/ml |
| Prolactin | 0.1-1 $\mu$g/ml |
| Choline chloride | 1-20 $\mu$g/ml |

Bovine Pituitary Extract (BPE) can be partially replaced by a combination of the following: Fibroblast Growth Factor (1-1000 ng/ml), phosphoethanolamine ($10^{-6}$-$10^{-3}$M), ethanolamine ($10^{-6}$-$10^{-3}$M), and Prostaglandin $E_2$ ($PGE_2$) ($10^{-10}$-$10^{-6}$M). Insulin can be partially replaced by Insulin-like Growth Factor (IGF-I), 10-1000 ng/ml. Hydrocortisone can be replaced by dexamethasone.

When the HFP cells are cultured in the serum-free medium, the surface of the culture vessel must be precoated with an extracellular matrix (ECM), preferably the extracellular matrix described hereinafter in Table C. For attachment and growth of HFP cells on uncoated tissue culture dishes, the medium must be additionally supplemented with serum such as 10% Fetal Bovine Serum (FBS).

The primary cultures established from the digested pancreatic tissue contain, in addition to pancreatic islet progenitor cells, other cell types including exocrine cells. The exocrine cells contain potentially damaging proteolytic enzymes, and it is optimal to additionally supplement the culture medium with detoxifiers, including a combination of the detoxifiers shown in Table B.

TABLE B

| Component | Concentration |
|---|---|
| Aprotinin | 1-10 $\mu$g/ml |
| Albumin (e.g., HSA) | 10-500 $\mu$g/ml |
| Catalase (activity about 50,000 units/mg) | 1-20 $\mu$g/ml |
| Superoxide Dismutase | 5-500 Units/ml |
| Glutathione | 0.05-20 $\mu$g/ml |
| Selenium | $10^{-8}$ to $10^{-6}$ M |
| PVP-360* | 0.1-1 wt.% |

*Polyvinylpyrrolidone (Sigma)

Aprotinin can be replaced with another suitable protease inhibitor, such as soybean trypsin inhibitor (1-100 $\mu$g/ml). HSA is added to absorb non-specific protease activity. Catalase, glutathione and selenium are involved in reactions which decompose peroxides. Superoxide dismutase decomposes superoxide radicals. The detoxification supplements are particularly important for serum-free primary culture conditions and after subculture and harvest when cell damage and death are occurring. Serum (e.g., 10% FBS) may partially substitute for the detoxification supplements. Prolonged exposure of the cells to serum (> 4 days) may, however, be inhibitory to HFP islet progenitor cell growth.

HFP islet progenitor cell growth is improved when the external gas environment contains higher than conventional levels of oxygen normally used for cell culture. A gas environment for HFP islet progenitor cells consists of a mixture of 30% oxygen, 65% nitrogen and 5% carbon dioxide.

TABLE C

| Component | Concentration |
|---|---|
| Laminin | 0.01-10 mg/ml |
| Collagen Type I | 0.01-4 mg/ml |
| Collagen Type IV | 0.001-2 mg/ml |
| Heparan Sulfate Proteoglycan (HSPG) | 0.001-2 mg/ml |

**EXTRACELLULAR MATRIX**

Optimal growth of HFP islet progenitor cells requires a suitable extracellular matrix (ECM). The following preferred ECM mixture can be coated on the surface of a tissue culture dish for monolayer cell culture, or, in an especially preferred embodiment, it can be mixed with cells to form a gel for growth of cells in a three-dimensional culture system. An optimal ECM mixture is shown in Table C.

Three-dimensional gels are an especially preferred environment for the implantation of fetal pancreatic progenitor cells. Epithelial cells require attachment to a support for optimum growth. Unlike many endothelial cells and fibroblast cells, most epithelial cells will bind only to a few specific substances under circumstances which are not fully under-stood. For both optimal cultivation and implantation, provision of a support to which the epithelial cell will firmly attach is a major requirement. Contraction of the hydrated gel (or hydrogel) structure to form more compact lattices is often desirable for cell culture and for implantation.

**SUBCULTURE OF PRIMARY CULTURES**

A pronase enzyme solution (0.05-0.1% pronase in HBSS - 0.15% HSA) is used to separate HFP epithelial cells from any contaminating fibroblasts in primary cultures. The HFP cell monolayers are first

6

rinsed with HBSS-CMF-0.15% HSA (HBSS without calcium or magnesium; human serum albumin added to 0.15%), and then treated with the pronase enzyme solution for 5 to 20 minutes at 37°C. Pronase treatment causes the epithelial cell patches to detach as sheets of cells which subsequently round up into multicellular clusters, whereas the fibroblasts detach as single cells. The detached cells are transferred to a 15 ml centrifuge tube containing wash medium such as basal medium M199 supplemented with antibiotics, 10 mM HEPES and 10% FBS.

The single cell fibroblasts are then separated from the larger epithelial cell clusters by low speed centrifugation (e.g., 2 min at 350-450 rpm). The fibroblasts are aspirated off with the supernatant. The cell pellet containing the large cell clusters is resuspended in wash medium and then centrifuged again. This step is repeated, then the cell pellet is resuspended in culture medium for the third wash step. After centrifugation, the epithelial cell clusters in the pellet are largely free of contaminating fibroblasts. The isolated HFP epithelial cells are then resuspended in culture medium and seeded into ECM-coated culture dishes. After proliferation in secondary culture, cells can be harvested for implantation or further subcultured for more expansion.

## FURTHER SUBCULTURE OR HARVEST OF PROGENITOR CELLS

Isolated single HFP progenitor cells subculture and transplant poorly. Therefore, the pronase enzyme solution is used to subculture HFP epithelial cell clusters for continued expansion, to harvest cells as multicellular clusters (10-2000 cells) for implanting, or for preparing biocompatible matrix implant products. The pronase procedure is the same as that used for isolating HFP epithelial progenitor cells from primary cultures. Care must be taken to prevent excess pipetting of the cell clusters during the procedure so as to minimize cell damage. If the cells are to be proliferated further, the epithelial cell clusters obtained from the pronase procedures are reseeded into ECM-coated culture vessels in serum-free culture medium. If the cells are harvested for transplant, cell viability and function (e.g., differentiation of structures with cells secreting insulin in response to secretagogues) is improved by suspension culture (up to 16 hours) of the harvested HFP epithelial cell clusters in HSA-coated dishes (to prevent cell attachment) in serum-free culture medium at 37°C in a humidified gas incubator (30% oxygen, 65% nitrogen and 5% carbon dioxide). During suspension culture, the HFP cell clusters round up into balls of cells and can be subsequently handled with minimal cell damage for implantation or incorporation into or onto biocompatible matrix implant systems.

The preferred contractible epithelial cell-collagen hydrogel compositions of this invention comprise a lattice structure which consists essentially of from 0.01 to 4.0 mg/ml collagen type 1, from 0.001 to 2.0 mg/ml collagen type IV, from 0.01 to 10.0 mg/ml laminin and at least $2 \times 10^7$ epithelial cells per ml. The uncontracted hydrogel is preferably in the form of droplets having a volume of from 2 to 1000 $\mu$L.

The gelation mixture is formed by mixing aqueous solutions of the collagen and laminin components and the cells in the proportions listed above, at a pH of from 6.5 to 8.5 and preferably from 6.8 to 7.6, and a temperature of from 2°C to 10°C and preferably from 2°C to 8°C. The mixture is then formed into droplets having the desired size. Adjusting the pH of the mixture to within the range of from 6 to 8 effects gelation, yielding a semisolid collagen hydrogel lattice incorporating the collagen type IV, laminin and epithelial cells. These droplets can be maintained in the uncontracted state by maintaining them at a temperature below 4°C for several weeks. The contraction is initiated by raising the gel droplet temperature above 25°C. Preferred contraction temperatures are within the range of from 30°C to 37°C. A humidified 37°C incubator may be used to facilitate the contraction process.

## PROLIFERATED HUMAN FETAL PANCREAS PROGENITOR CELL CHARACTERISTICS

Human cells can be distinguished from animal cells that produce insulin by an assay specific for human C-peptide. *In vitro* or *in vivo* quantification of human C-peptide can be performed on cell culture fluids or on serum samples taken from transplant recipient.

Fetal cells can be distinguished from adult cells by histologic examination of the cells, noting the degree of fibrosis and structural organization. Immunostaining reveals the number of hormone positive cells. Islets derived from adult pancreatic tissue have a much higher percentage of hormone-containing cells and less stromal elements than cell clusters derived from fetal tissue. Electron microscopic examination

7

provides a confirmatory or backup test for the presence of mature and immature pancreatic islet cells as well as exocrine lineage cells in tissues.

Proliferated cell products of this invention have characteristics which can readily distinguish them from non-proliferated products. Proliferated cells of this invention can be distinguished from non-proliferated tissue pieces, collagenase-digested units (CDU's) and minimally proliferated cultured cells on the basis of the following examinations:

A. Proportion of islet cells

The prior art collagenase digest units (CDU's) and minimally proliferated HFP cells are enriched in immature islet cells.

Fedorov et al. (supra) report achieving cell colonies with limited fibroblast contamination with from 60 up to 90 percent immature $\beta$-cells. The proliferated progenitor cell cultures of this invention contain 5% or less total pancreatic islet cells. Sandler et al., *Diabetes* 34:1113-1119 (1985) and Kohnert et al., *Acta Biol.Med.Germ.* 41:1163-1170 (1982) report having put CDU's into culture in serum and show that fibroblasts will grow out on a monolayer from the CDU's, leaving the immature pancreatic islet cells behind in small clumps or colonies. The colonies are detached from the monolayer of the fibroblasts by gentle pipetting. In these culture systems, there appears to be no increase in the yield of pancreatic islet progenitor cells or of immature pancreatic islet cells over the original number in the pancreatic tissue. Rather, the enrichment for pancreatic islet cells results from depletion (loss) of contaminating cell types (i.e., fibroblasts by outgrowth from the tissue (McEvoy et al., *Med.Biol.* 64:271-276 (1986)) and possibly exocrine cells by necrosis (Mandel, et al., *Diabetes* 31 (Supp. 4):31-47 (1982); Agren et al., *Diabetes* 29:64-69 (1980); Hellerstrom et al., *Diabetes* 28:769-779 (1979)). There appears to be little, if any, selective proliferation of immature pancreatic islet cells or pancreatic islet progenitor cells.

The proliferated cells of this invention are produced by processes which are optimized for proliferation of human fetal pancreatic islet progenitor cells. Immature pancreatic islet cells (which are committed to differentiation) have a limited proliferative capacity *in vitro* and *in vivo* (after implant) as reported by Hegre et al., *Acta Endicrin.* 83 (supp.205):257-278 (1976). As a result, in the culture methods of this invention, the immature pancreatic islet cells decrease in percentage of the total population with increasing time of culture. Whereas immature pancreatic islet cells usually approximate 10 to 20 percent in a CDU preparation from HFP, this number is usually decreased to five percent or less in the proliferated pancreatic islet progenitor cell compositions of this invention.

B. Proportion of fibroblasts in the harvested cell population:

The percentage of fibroblasts in non-proliferated HFP is determined by the method of preparation. If one produces tissue blocks by simply chopping tissue into small (1-2 mm) pieces and cultures them as tissue explants, fibroblasts are a major contaminant (50 percent or greater). This is a result of no separation of epithelial cells from the stromal or fibroblast cells. Fibroblasts survive and readily proliferate in explant culture, far better than epithelial cells, and often form dense fibrous capsules around the tissue block (Mandel, et al., *Diabetes* 31 (Supp. 4):31-47 (1982); Agren et al., *Diabetes* 29:64-69 (1980); Murrell, *Exp.Cell Res.* 41:350-364 (1966)). In contrast, epithelial cells tend to decrease in number with time in culture as evidenced by an increase in cell necrosis.

CDU's are prepared by treating HFP tissue with collagenase. This process removes a large amount of the fibroblastic stromal tissue, leaving small clusters of predominately epithelial cells (CDU's). If the CDU's are allowed to attach to a culture surface such as a tissue culture dish in the presence of serum, fibroblasts will preferentially grow out from the cell clusters. Epithelial cells remain behind, as indicated previously, and can be harvested by gentle pipetting. The proportion of fibroblasts in these detached cell clusters will vary, but may be as little as 10 percent of the population.

In the proliferated cell product of this invention, the fibroblasts are largely eliminated and make up less than about 10 percent of the harvested cell population, preferably less than about 8 percent, and more preferably less than about 6 percent of the harvested cell population.

C. Transplantation Assay:.

1) Grafts in nude mice of proliferated cells made according to the procedure of Example 6 (hereinafter) can be distinguished from grafts of HFP tissue pieces or blocks (BDU's) on the basis of degree of fibrosis, proportion of exocrine cells and proportion of pancreatic islet progenitor cells to pancreatic islet cells.

Examining the degree of fibrosis of the implants, BDU grafts are usually large and fibrotic. Fibroblasts make up a major proportion of the implant material and will proliferate in the graft. The proliferated pancreatic islet progenitor cell grafts of this invention are smaller and usually have only a small amount of fibrosis. Since fibroblasts make up less than 10 percent of the original- cell population with the cell implants of this invention, most of the fibrosis occurs as a result of the surgical procedure.

Examining the proportion of exocrine cells, BDU grafts often contain a large mass of exocrine tissue, as distinguished by their characteristic morphology. Exocrine cells are a contaminating cell type in the original tissue and will proliferate in the graft, as indicated by the large increase in their mass. The proliferated cell implants of this invention-rarely contain any significant amount of exocrine cells.

Since BDU grafts contain fibroblasts and exocrine cells in addition to immature pancreatic islet cells, immature pancreatic islet cells make up a fraction (usually 10 to 20 percent) of the total graft mass. The proliferated cell grafts of this invention contain predominately (greater than 50%) pancreatic islet cells and islet progenitor cells, the latter forming tubular structures in the graft.

2) Grafts of the proliferated cell compositions of this invention in nude mice or other rodent models can be distinguished from non-proliferated BDU's and CDU's, and cell clumps harvested from fibroblast cultures of CDU's on the basis of the time for maturation cf pancreatic islets post-transplant and the time required to cure a diabetic mouse.

The time for maturation of pancreatic islets post-implant is examined histologically and functionally. CDU's and their primary culture derivatives, which contain immature pancreatic islet cells (the degree of islet maturity depending upon the gestational age of the donor), provide mature pancreatic islets in a shorter time period after implant than the proliferated pancreatic islet progenitor cells of this invention. The proliferated cells of the composition of this invention will continue to proliferate after implantation, and must first develop into immature pancreatic islet cells before maturing into functional pancreatic islets capable of secreting insulin in response to glucose.

Examining the time required to cure a diabetic mouse, the implantation of BDU or CDU products will provide a decrease in blood glucose level in the mouse to within normal levels by about 60-90 days following implantation. After implantation of the proliferated pancreatic islet progenitor cells of this invention, a longer developmental time is required before mature, functioning pancreatic islets develop, and blood glucose levels are not normal at 100 days following implantation. As shown by Figures 1 and 2, blood glucose levels do not usually reach normal levels until about 110 to 120 days following implantation.

As can be seen by the above, the proliferated cell compositions of this invention are uniquely different from the previously developed and implanted adult and fetal pancreatic cultures, compositions and implants. The ability to proliferate HFP islet progenitor cells results in a greatly improved source of implant cell compositions for treating diabetic conditions wherein the patient has a reduced or nonexistent pancreatic $\beta$-cell function.

This invention is further illustrated by the following specific, but non-limiting examples. Unless otherwise specified, all temperatures are in degrees centigrade and all percentages are in weight percents. Procedures which have been reduced to practice are presented in the past tense, and procedures and products which are first reduced to practice in the filing of this application are presented in the present tense.

## EXAMPLE 1

### Tissue Collection

The pancreas was aseptically dissected from the fetus and placed in a sealed container with cold (0-8° C) shipping medium so that processing of the tissue could take place at a later time. 4 suitable medium is L-15 (Liebovitz) medium supplemented with 5% fetal bovine serum (FBS) and antibiotics. The optimal time lapse between dissection and commencement of processing for culture is less than 4 hours. Times longer than this result in cold ischemia and loss of cell viability. Cold ischemia times of 24 hours or more results in a 50-95% loss of cell viability.

The contents of the shipping container were poured into a cold, sterile dish. All steps were done under

9

a Laminar Flow Hood using aseptic technique. A pair of sterile forceps was used to dissect away any residual connective tissue or membrane.

## EXAMPLE 2

### *Tissue Preparation*

The cleaned pancreas of Example 1 was transferred to a sterile 6 ml tube containing 2.0 ml of HBSS supplemented with 0.15% HSA and 10 mM HEPES (HBSS-HSA). Each pancreas was so processed, and labeled.

Using sterile forceps, a pancreas was transferred from the 6 ml tube and onto the chopping platform of a tissue chopper (McIllwain) which was wiped with 70% ethanol and placed in a Laminar Flow Hood under UV light and blower for at least 30 minutes prior to use. The specimens were chopped into 1mm pieces. After the first series of chops, the platform was rotated 90° and the specimen was chopped again. Each pancreas was chopped separately, changing the razor blade and plastic chopping platform between each pancreas.

The tissue blocks were transferred from the chopping platform using a 10 ml pipet containing 8.0 ml of Collagenase Enzyme Solution. The Collagenase Enzyme Solution contains: 0.75 mg/ml Sigma Collagenase Type XI and 0.1 mg/ml Sigma DN'ase in HBSS-HSA. Approximately 0.5 ml of the Collagenase was released to suspend the tissue blocks and as much of the tissue as possible was aspirated into the pipet. This was transferred to a suitable digestion vessel, e.g., a 25 ml Erlenmeyer flask. All except 2-3 ml of the Collagenase was released from the pipet. Any residual tissue from the chopping platform was aspirated in another 0.5 ml of Collagenase, and transferred by pipet to the flask. The flask was then capped tightly, and labeled.

## EXAMPLE 3

### *Preparation of the Primary Culture*

The flask containing chopped pancreas produced by the methods of Example 2 was incubated for 15-20 minutes at 37°C in a reciprocal shaking water bath, then removed, and the contents triturated 4-6 times to further disperse the tissue. The large tissue pieces were allowed to settle (20-60 seconds). Tissue digestion is complete when one or more of the the following conditions exist: the digest supernate is no longer turbid; there are no large tissue pieces remaining; the tissue fragments do not decrease in size with up to a maximum of five digests.

The supernate was pipetted from the flask with a sterile pipet, being careful not to transfer any large tissue pieces, and transferred to a 15 ml centrifuge tube (wash tube) containing 4 ml of Wash Medium. A suitable wash medium is M199 supplemented with mM HEPES, 10% FBS and 0.1% gelatin. If there were large tissue pieces remaining, 4.0 ml of fresh Collagenase Enzyme Solution was added to the flask, with additional incubation on the shaker bath of 10-15 minutes. Digestion was continued until the tissue was completely digested. Additional digest products were prepared for centrifuging as above.

The wash tube(s) containing the supernate from the digest were centrifuged at 700 to 950 rpm for 3 minutes. The supernate(s) were aspirated using sterile Pasteur pipet(s).

The cells were resuspended in 10 ml of Wash Medium, and centrifuged for 3 minutes at 700-950 rpm. The supernate was aspirated. The cells were resuspended in wash medium and centrifugation and aspiration were repeated.

The cell pellet was resuspended in 10 ml of Culture Medium. A suitable culture medium is medium M199 supplemented with 10 mM HEPES, antibiotics, and the following:

## TABLE D

| Culture Medium<br>Hormones and growth factors | Source | Final<br>Concentration |
|---|---|---|
| Bovine Pituitary Extract (BPE) | Dept. Mol.<br>& Cell Biol.<br>U of Colorado<br>Boulder, CO. | 10 µg/ml |
| Insulin | Sigma | 5 µg/ml |
| High Density Lipoprotein (human) | Hana | 50 µg/ml |
| Growth Hormone | Schwartz-Mann | 0.5 µg/ml |
| Glucagon | Elanco | 0.5 µg/ml |
| Epidermal Growth Factor | Coll. Res. | 25 ng/ml |
| Triiodothyronine ($T_3$) | Sigma | 0.1 ng/ml |
| Hydrocortisone | Sigma | 0.5 µg/ml |
| Prolactin | Sigma | 0.1 µg/ml |
| Choline Chloride | Sigma | 10 µg/ml |

## TABLE D (contd)

| Culture Medium<br>Detoxification supplement: | Source | Final<br>Concentration |
|---|---|---|
| Aprotinin | Canada Packers | 3 µg/ml |
| Catalase | Sigma | 5 µg/ml |
| Superoxide dismutase | Sigma | 50 Units/ml |
| Selenium | Sigma | 10 ng/ml |
| Human serum albumin (HSA) | Miles | 0.25 mg/ml |

For primary culture, 10% FBS was also added. For secondary culture (see below), the cells were seeded in Culture Medium supplemented with 10% FBS, but the medium was changed to serum-free conditions at 24 hours after seeding.

The cells were seeded into 100 mm tissue culture dishes at $2\text{-}3\times10^6$ cells/dish. If necessary, the volume of Culture Medium in the dishes was adjusted to 5.0 ml. Cultures were incubated at 37°C in an atmosphere of 30% $O_2$ 5% $CO_2$, and 65% nitrogen. The cultures were fed on the day after seeding with fresh Culture Medium containing 10% FBS.

## EXAMPLE 4

### Subculture of the Primary Culture

After 4 days of primary culture, the cultures contain epithelial cell patches surrounded by contaminating fibroblasts. At this stage they are subcultured to remove fibroblasts.

The medium was aspirated from each dish using a sterile Pasteur pipet for each specimen. Each dish was rinsed with calcium- and magnesium-free HBSS supplemented with 10 mM HEPES and 0.15% HSA

(HBSS-CMF-HSA). Rinse medium was aspirated off using a sterile Pasteur pipet, and each dish was rinsed again with HBSS-CMF-HSA. The rinse medium was again aspirated using sterile Pasteur pipets.

Pronase is a suitable enzyme for subculture of primary culture because it will detach the epithelial cell patches more or less intact, whereas the fibroblasts detach as single cells. 2.0 ml of 0.05% Pronase Enzyme Solution (0.05% pronase in HBSS-HSA) was added to each dish using a sterile pipet. Each dish was then incubated for 7 ± 5 minutes in a 37°C humidified gas incubator. Each dish was then examined under a phase contrast microscope to see if all the cell patches had detached from the culture surface. If they had not detached, the dish was reincubated for an additional 3 to 5 minutes.

If less than 90% of the cell patches had detached, the enzyme solution was pipetted using a 5 ml pipet that had been pre-coated with HBSS-HSA, and loose cell patches were gently washed off. The enzyme solution containing released cells was then transferred by pipet to a 15 ml centrifuge tube. Wash Medium was added to the tube, to a final volume of 10.0 ml. Additional 0.05% Pronase Enzyme Solution was added, and the tube was incubated for 7±5 minutes in a 37°C humidified gas incubator, and processing was continued as follows.

After more than 90% of the cell patches had detached, the cells from up to 3 dishes of the same specimen were collected in 6.0 ml of Wash Medium. The medium was transferred from dish to dish, then transferred to a 15 ml centrifuge tube. The dishes were rinsed with an additional 4.0 ml of Wash Medium, and the rinse was placed in the same centrifuge tube.

Each tube was centrifuged for 2 minutes at 300 to 500 rpm, and the supernate was aspirated. The cell pellets were resuspended in Wash Medium. At this time, cells from separate dishes of the same specimen may have been pooled into one or two centrifuge tubes. The tubes were centrifuged for 2 minutes at 300 to 500 rpm, and the supernate was aspirated. The cell pellet was resuspended in 10.0 ml Wash Medium. The centrifugation and wash steps were repeated twice.

The final cell pellet was resuspended in Culture Medium containing 10% FBS. Cells were seeded into ECM-coated 100 mm dishes at approximately 2-3x10$^6$ cells/dish. (To coat the dishes with ECM prior to seeding, place the tissue culture dishes on a tray of ice and add 2-3 ml/dish of a cold solution containing: 0.25 mg/ml Collagen Type I; 0.01 mg/ml collagen Type IV; and 0.35 mg/ml Laminin in M199. Spread the solution completely and evenly over the bottom of the dish. Allow to stand 2-5 minutes on ice, and then aspirate. A residual film of ECM will remain on the dish.)

The secondary cultures were fed 24h after subculture with serum-free Culture Medium, washing once with HBSS-HSA prior to adding fresh medium. The cultures were fed with fresh serum-free culture medium every second day thereafter.

## EXAMPLE 5

### Harvest and Packaging Cells for Transplantation

Cells of Example 4 were harvested as cell clusters for transplant using the same pronase procedure as for subculture of primary cultures. The harvested cells were seeded into HSA-coated 100mm dishes in serum-free Culture Medium. The suspension cultures were incubated at 37°C in a humidified incubator (in an atmosphere of 30% $O_2$, 5% $CO_2$, and 65% nitrogen) for 16-24 hr, which allows the cell clusters to round up into tight aggregates prior to packaging.

After suspension culture, harvested cells were pooled (e.g., according to ABO typing), and then divided into aliquots of $10 \times 10^6$ cells per 15 ml centrifuge tube in 10 ml of serum-free Culture Medium.

A suitable packaging mixture contains 1.65 mg/ml Collagen Type I, 0.35 mg/ml Laminin; 0.01 mg/ml Collagen Type IV. The pH was adjusted to 7.0-7.5 with NaOH. Tubes of packaging mixture and cells suspended in packaging mixture must be kept in wet ice before aliquoting the cells into beads. Packaging mixture, cells and gelled beads were handled aseptically in a Laminar Flow Hood throughout all steps. The culture media must be at room temperature.

For $10 \times 10^6$ cells, 0.25 ml of packaging mixture was added. If there were more or fewer cells in the tube, the amount of packaging mixture·was adjusted with the following formula:

(ml packaging mixture needed) = (cell number/$2 \times 10^5$)(0.005 ml)

The tube of cells was centrifuged at 600 rpm for five minutes, and the supernatant was aspirated. Without resuspending the pellet, the tube was spun again at 400 g by bringing the centrifuge rapidly up to 1600 rpm, then immediately returning time and speed controls to zero.

Any residual medium above the pellet was carefully aspirated with a Pasteur pipet. The tube containing the cell pellet was placed in a container of ice in the Laminar Flow Hood..The amount of packaging mixture determined for that tube was transferred: without changing tips of pipets, the cell and packaging mixture were mixed together by gently pipetting up and down. Bubbling of the mixture and cells was avoided.

A clean automatic pipet capable of dispensing 5 µl aliquots was used to aspirate up the solution with the cells suspended in packaging solution. The tip of the pipet was gently touched to the inside bottom of a sterile petri dish and 5 µl aliquots of cells plus packaging mixture were pipetted out to form a series of beads. This was repeated until all packaging mixture and cells have been delivered to the petri dish. Bead preparation was conducted quickly to prevent the mixture for gelling prematurely in the pipet tip.

The petri dish was placed carefully into a 5% $CO_2$ humidified incubator at 37°C for 30 minutes or until gelled.

Using a 10 ml pipet, the dish of gelled beads was flooded with 20 ml of serum-free Culture Medium. The gelled beads were gently detached from the bottom of the dish with a pipet. At this stage, the individual beads can be easily handled for transplantation.


**EXAMPLE 6**


*Experimental Protocol for Evaluating*
Proliferated Cells


Tissue Preparation:

1. Render the tissue, either cultured or uncultured, aggregates or minced pieces, approximately the same size (75-200 microns in diameter). Non-proliferated tissue should be either finely minced or collagenase digested into small fragments before being placed into culture.

2. Transplant the non-proliferated or proliferated tissue within
48 hours of being placed in culture, or of being harvested.


Preparation Immediately Prior to Transplant:

3. The tissue is to be implanted under the renal capsule, in non-diabetic nude mice. Tools, anesthetics, and supplies are procured and laid out prior to the surgery.

4. Procure human plasma using an EDTA vacutainer tube (lavender top). Do not use a heparanized tube. Separate the plasma and hold on ice until transplant (not more than 5 hours).

5. Place four aliquots of 30-50 aggregates or tissue fragments in wells of a 96 well plate. They should be in a culture medium such as M199.

EP 0 363 125 A2

6. After the mouse is anesthetized and before surgery is commenced, draw off the medium from the tissue. To each well containing tissue, add 10 μl of human plasma. Then add to each well, 5 μl calcium chloride (sterile, 0.01 M). The plasma should have formed a clot around the tissue by the time the kidney has been exposed and prepared for transplant.

7. Remove the clots from the well by first teasing them away from the wall of the well using forceps. Transfer the clots to the prepared mouse kidney and implant them under the renal capsule near the kidney pelvis. The clots containing tissue should be positioned close to each other, but not on top of each other.

8. After implantation, the mouse is kept for thirty days. At thirty days, the kidney, containing the graft, is removed and submitted for histology.

Histology:

9. The kidney is fixed in formalin. The part containing the graft is paraffin embedded and sectioned. Stains that should be done include Hematoxylin and Eosin, and immunostains for glucagon, insulin and somatostatin.

Evaluation:

10. Grafts of proliferated pancreatic islet progenitor cells can be distinguished from grafts of non-proliferated cells on the basis of degree of fibrosis, proportion of exocrine cells, and proportion of pancreatic islet progenitor cells to pancreatic islet cells, as described above.

**Claims**

1. A mixture containing proliferated human fetal pancreatic islet progenitor cells, the proportion of pancreatic islet progenitor cells to exocrine and fibroblast cells being substantially greater than in fetal pancreas tissue.

2. The mixture of Claim 1 comprising a nutrient culture medium containing proliferated human fetal pancreatic islet progenitor cells.

3. The mixture of Claim 1 wherein the proliferated human fetal pancreatic islet progenitor cells are in contact with a substrate packaging matrix.

4. The mixture of Claim 3 wherein the substrate matrix includes
Collagen Type I.

5. The mixture of Claim 4 wherein the substrate matrix is a contracted matrix containing Collagen Type I, substantially free from fibroblast cells.

6. The mixture of Claim 5 wherein the matrix contains laminin and
Collagen Type IV.

7. The mixture of Claim 3 in the form of a droplet suitable for implantation.

8. The mixture of Claim 7 wherein the matrix is a contracted matrix containing Collagen Type I, substantially free from fibroblast cells.

9. The mixture of Claim 8 wherein the matrix contains laminin and
Collagen Type IV.

10. A process for preparing proliferated human fetal pancreatic islet progenitor cells comprising

(a) culturing human fetal pancreatic tissue containing progenitor cells to decrease the number of fibroblast cells and exocrine cells; and

(b) proliferating to increase the number of islet progenitor cells.

11. A process of Claim 10, further comprising

a) proliferating a primary culture of human fetal pancreatic cells;

b) separating human fetal pancreatic epithelial cells including pancreatic islet progenitor cells from human fetal pancreatic fibroblast cells by subculture; and

c) proliferating the epithelial cells through one or more subculture until the relative proportion of the pancreatic islet progenitor cells to fibroblast and exocrine cells and the total number of pancreatic islet progenitor cells is greater than in the original fetal pancreas.

12. A process of Claim 10 further comprising including proliferated pancreatic cells in a packaging matrix.

13. Proliferated human pancreatic progenitor cells.

14